# EUROPEAN PATENT APPLICATION

(11) **EP 1 327 422 A1**
(43) Date of publication of application: **16.07.2003**
(21) Application number: 03394003.2
(22) Date of filing: 07.01.2003
(51) Int. Cl.: A61F 2/06

(54) **Stent delivery system**

(30) Priority: 14.01.2002 US 50651
(71) Applicant: Medtronic AVE, Inc., Santa Rosa, California 95403 (US)
(72) Inventor: Gallagher, Brendan P., Galway (IE); Rogers, Ronan, Galway (IE); Gribbons, Richard, Galway (IE)
(74) Representative: McKeown, Yvonne Mary

(57) **Abstract**

The present invention provides a medical stent delivery device (40) for delivering a stent to a treatment site. The device has an elongated catheter with an inflatable balloon (44) attached to its distal end for receiving a stent which is expandable radially upon inflation of the balloon. The elongated catheter includes a transitional area of material (41, 416) proximal and/or distal to the stent.

## Description

### Field of the Invention

This present invention relates to a vascular catheter, and more particularly to a catheter which is used for the delivery of a therapeutic to a body vessel.

### Background of the Invention

Vascular catheters are used in a variety of therapeutic applications, such as the delivery of stents for intraluminal endovascular stenting and other stenting applications. Such a stent acts *in situ* as a scaffolding and support prosthesis for a damaged or collapsed vessel wall. Percutaneous transluminal coronary angioplasty (PTCA) is used to clear coronary arteries which have become occluded by a build-up of cholesterol fats and/or artherosclerotic plaque. Typically a guide wire is steered through the vascular system to the site of therapy and a balloon catheter is advanced over the guide wire. The balloon at the distal end of the catheter is inflated at the diseased location causing the site of stenosis to widen. The expansion of the vessel can form nuances, which threaten re-closure of the vessel or perforations in the vessel wall. Implantation of a stent can provide support for the vessel wall and hence help to avoid reclosure of the vessel or can provide a temporary repair patch until corrective surgery can be undertaken. Stents can also be used to repair aneurysms, to support artificial vessels as liners of vessels or to repair dissections.

A stent typically is a cylindrical shaped device formed from metal or polymers. It can be permanently placed within a vessel to hold the lumen open, thus reinforcing the lumen and improving blood flow. A stent generally is radially compressed to a diameter that is smaller than that of the vessel in which it is to be deployed to enable it to be delivered to the treatment site. Once the stent has been delivered to the treatment site, it is expanded radially to allow it to contact and support the vessel wall. Some stent types are fabricated to elastically resist compression in a free state, and are releasably compressed for delivery. A second type is the radially expandable stent, which is crimped onto an expansion device such as a balloon catheter for delivery and expanded radially at the treatment site.

In the latter case, the stent is crimped onto a balloon, introduced percutaneously, transported transluminally and deployed at the treatment site by inflation of the balloon. A balloon capable of withstanding relatively high inflation pressures is preferable for stent deployment, as it is very important that the stent fully expands. If the stent does not fully expand, it may hang down in the vessel and promote the formation of thrombi or difficulties in introducing treatment catheters past the not fully expanded stent, which is undesirable. Additionally, the vessel may not fully expand and thus the vessel is not optimally treated.

The invention described herein is equally applicable to both stent types described above.

Numerous stent devices and delivery systems are known. US Patent No. 5,776,161 issued to Globerman discloses a stent which is substantially flexible along its longitudinal axis when constrained, allowing it to move with ease through tortuous body vessels to the treatment site. However despite this, it is known to those skilled in the art that in the course of the delivery of a stent to a treatment site by a balloon catheter the device may kink or hinge as the stent is eased through vessels having tortuous narrow acute angles. This hinging effect can have further consequences, as it may cause the balloon on which the stent has been crimped to stretch on the outer radius of the tortuous vessel and bunch on the inner radius of the tortuous vessel. This can create a gap between the balloon and stent, which exposes the leading or trailing edge of the stent thus exacerbating the difficulty of traversing the vessel tortuosity, and introducing the risk that the relatively stiffer stent edge may tear or damage the vessel wall. The stent may become longitudinally displaced on the balloon interfering with deliverability and proper full deployment of the stent. Additionally, other types of vascular procedures have devices delivered via a flexible catheter. These include devices for radiation treatment, drug delivery and other treatment and diagnostic devices. These devices can also be prone to kinking or hinging intermediate the treatment device and the flexible catheter adjacent the treatment device.

A variety of approaches have been taken to address this issue, and in particular, focus on the interface of the stent and the balloon. WO 9612517A1 and European Patent Applications Nos. 1 000 591, 1 000 592 and 1 000 593 disclose a balloon catheter and stent delivery system with enhanced stent retention and a method for making a balloon catheter and stent delivery system with enhanced stent retention, thereby addressing a number of issues associated with stent delivery. The use of balloon pillows within the interstices of the stent and pillows proximal and distal to the stent for retaining the stent in position is disclosed. WO 98/07390 discloses another approach to enhanced stent retention. This discloses a variety of configurations of a coaxial member within the balloon that is designed and adapted to provide a securement pressure to the stent in the delivery diameter to maintain the stent in position on the catheter during delivery to the deployment site. However additional improvements in stent delivery are still required to address the difficulties mentioned above. In particular, this does not address the difficulties of hinging or kinking that occur between the stent and the catheter adjacent the stent.

It is an object of the present invention to seek to alleviate the aforementioned problems.

### Brief Summary of the Invention

Accordingly, the present invention provides an intravascular catheter comprising a relatively flexible delivery means adapted to retain a relatively stiff stent, the device including a region of material located at a position on the delivery means approximate the stent which has a degree of stiffness intermediate those of the delivery means and the stent.

In particular, the present invention provides a medical stent delivery device for delivering a stent to a treatment site. The device comprises a catheter having an elongated shaft having an outer and inner shafts having proximal ends and distal ends. An inflatable balloon is attached to the distal ends of the shafts for receiving a stent which is expandable radially on inflation of the balloon. The shafts have a relatively lesser degree of stiffness and the stent having a relatively greater degree of stiffness, characterised by the provision of an area of material proximal and/or distal to the stent of a degree of stiffness intermediate those of the shafts and the stent.

The present invention is suitable for use with many balloon catheters including the type having a balloon positioned at the distal end of a catheter. However the invention is not limited to this type of balloon catheter. The invention is also not limited to use with balloon catheters but can also be effective in assisting delivery of stents which self-expand on deployment and other vascular catheters delivering devices for other intravascular procedures as discussed earlier. In a typical arrangement, the device is a balloon catheter which has an inner shaft extending through an outer shaft and balloon attachment, the inner shaft protruding slightly distal of the balloon. The proximal end of the balloon is secured to the outer shaft and the distal end of the balloon is secured to the inner shaft. Typically, there are two radiopaque marker bands situated on the inner shaft distal and proximal to the stent for enabling the medical team to visualise and delineate the precise position of the stent within the patient's body.

In one embodiment of the invention, the catheter has one or more short sections of material of increased stiffness relative to the relatively flexible catheter shafts added to the inner shaft between the balloon distal end and the distal end of the stent and/or between the balloon proximal end and the proximal end of the stent. However the short sections of relative stiffness are not as rigid or stiff as the stent itself. In a convenient arrangement the area of relative increased stiffness is situated on the inner shaft so as to surround and overlie the marker bands. Conveniently the area of relative increased stiffness protrudes slightly under the stent at the proximal and distal ends of the stent. The areas of relative increased stiffness are placed in this position in order to smooth the transition from the flexible catheter to the less flexible stent. This reduces the risk of tracking and efficiency discontinuities which can result in the catheter kinking or hinging.

Ideally the areas of increased stiffness are provided by one or more transition sleeves placed about the inner shaft. In such case, the internal diameter of the sleeve is great enough to allow the sleeve to pass over the marker bands located on the inner shaft, and small enough to permit the sleeve to pass through the internal diameter of the balloon bond or weld for assembly of the delivery system.

Preferably, each sleeve is cut to length from a full-length extrusion of the required material extrusion. For exemplary purposes, one suitable material is 100% 7233 clear Pebax™ extrusion and many others will suggest themselves to the skilled person. The sleeve dimensions chosen will be dependent on the stent and stent delivery system being used. Ideally once the sleeves are cut, they are placed in position over the marker bands at the proximal and distal ends of the stent. Each sleeve is then fixed onto the inner shaft by lasering or any other suitable means. It is preferable for each sleeve to be fixed onto the inner shaft individually. Ideally, the sleeve material is transparent or translucent. This ensures that when the sleeve is fixed over a marker band, the band remains visible, which visibility facilities correct assembly of the delivery system. Alternatively, the sleeve may incorporate radiopaque material and operate as a marker also.

In an alternative arrangement of the invention no separate additional material or sleeve is fixed to the inner shaft to increase the relative stiffness of the inner shaft, but instead, the marker bands are designed to serve the dual purpose of increasing the stiffness proximally and/or distally to the stent.

In a further arrangement of the invention a section of the inner shaft intermediate the proximal and distal balloon welds or bonds (which may be made by suitable means including via heat, laser or an adhesive) is formed such that it is more rigid than the rest of the inner shaft, but not as rigid as the stent itself. Again, this arrangement provides an increase in the relative stiffness of the inner shaft proximally and distally to the stent. If present, the marker bands may be formed integrally with this section or may be applied to it.

### Brief Description of the Drawings

The invention will hereinafter be more particularly described with reference to the accompanying drawings, which illustrate by way of example only an embodiment of a stent delivery system according to the invention.

In the drawings:

Figure 1 is an elevational view of the distal end of a known balloon catheter stent delivery system;

Figure 2 is an elevational view of the balloon portion of a balloon catheter stent delivery stystem according to the invention with transition sleeves present;

Figure 3a is a view of a balloon catheter stent delivery system without transition sleeves exhibiting instability as it is positioned through an acute angle;

Figure 3b is a view of a balloon catheter stent delivery system with transition sleeves exhibiting a more stable bend as it is positioned through an acute angle.

Figure 4 is a 90° fixed wire track profile for a prior art stent delivery system.

Figures 5 and 6 are track profiles for delivery system for two stent sizes showing in each case the profile obtained with and without a transition sleeve.

### Detailed Description of the Invention

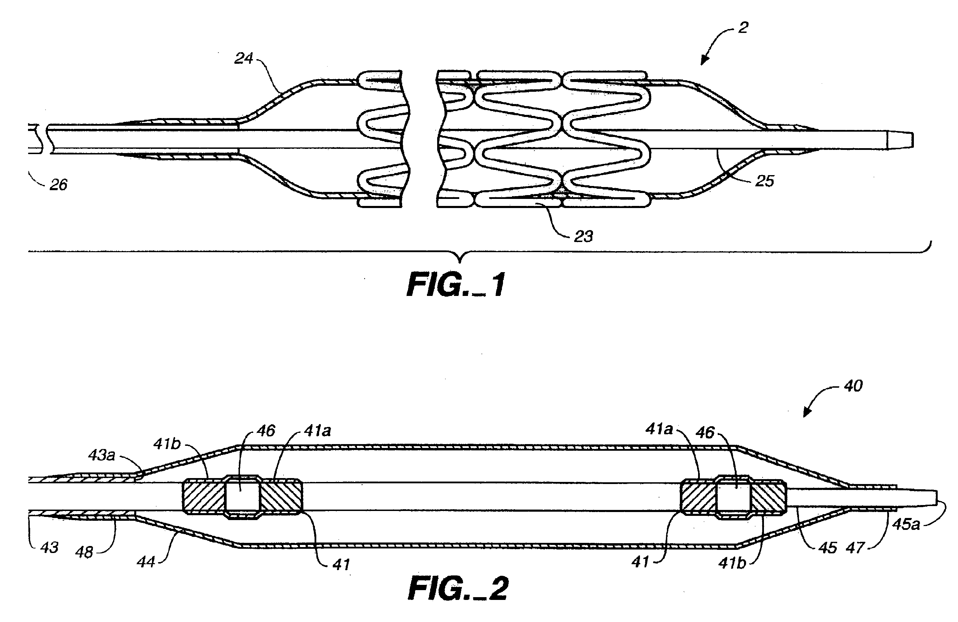

Referring to the drawings, Figure 1 is a detailed view of the distal end of a typical balloon catheter stent delivery system 2. As used herein, the terms distal and proximal refer to the balloon end and luer fitting end respectively. The device includes an inner catheter shaft 25 and an outer catheter shaft 26. A balloon 24 is welded at its distal end to the outside of the inner shaft 25 and at its proximal end to the outside of the outer shaft 26, and a stent 23 is assembled onto the balloon 24. The balloon 24 can be any balloon suitable for angioplasty procedures or any other stent delivery systems. A preferred type of balloon 24 is one with a plurality of folds (not shown) such that on inflation of the balloon, the stent is caused to expand radially in an even manner. The stent 23 shown is of the type having a hollow cylindrical body composed of a plurality of linked circumferentially extending rings. Other forms of stents and delivery systems for other intravascular procedural or diagnostic devices will equally be suitable for use with the device of the invention.

Figure 2 is an elevational view of the distal portion of a stent delivery system 40 according to the invention, with the stent omitted for clarity. The distal end includes a balloon 44 bonded at distal balloon bond or weld 47 to the outer surface of the inner shaft 45 and at proximal balloon bond or weld 48 to the outer surface of the outer shaft 43. In use, a guidewire (not shown) is extended through the lumen L of the inner shaft 45 and projects beyond its distal terminus 45a to help guide the delivery system to the desired treatment site. Located on the outer surface of the inner shaft 45 is a pair of marker bands 46 which underlie the balloon 44. The inner shaft 45 also includes a pair of transition sleeves 41 fabricated of a material to provide increased stiffness relative to the shafts 43, 45 and balloon 44 and of diminished stiffness relative to a stent mounted on the balloon.

One sleeve 41 is positioned over the inner shaft 45 beneath the balloon adjacent the distal balloon weld 47 and a second sleeve 41 is positioned beneath the balloon 44 and adjacent the proximal balloon weld 48. In an ideal arrangement, a stent positioned on the balloon 44 will be located to overlie the space between the transition sleeves 41 and will partially overlap a transition sleeve at each of the ends of the stent. The marker bands 46 are a typical feature of stent delivery systems. They are usually formed of a radiopaque material and are used by the physician to enable the position of the stent in a body vessel to be visualised. Whilst the stent has been omitted from this diagram for clarity, if it were in position it would be crimped over the balloon 44 between the marker bands 46. In this embodiment of the present invention, the sleeves 41 comprise lengths of tubing fixed in position on the inner shaft 45 above and overlapping the marker bands 46. The material selected is of a stiffness intermediate those of the relatively stiffer stent and the relatively flexible catheter shafts 45, 43. Once a stent is assembled onto the balloon 44, it rests between the pair of marker bands 46 so that the bands are clearly visible to the eye and are not rendered obscured to a non-visual imaging system. The visibility of the marker bands assists the assembly person in locating the stent at a correct position on the balloon intermediate the two marker bands and for this reason, it is desirable that the sleeve material should be transparent or translucent so that the bands remain visible beneath the sleeves. A first portion 41a of each sleeve is provided to underlie the stent and a second portion 41b is disposed clear of the stent so as to ensure as even as possible a transition in stiffness from catheter shaft to balloon to stent. The purpose of these stiffness transitions is to smooth out the stiffness profile of the combined balloon catheter and stent thus reducing the risk of tracking and efficiency discontinuities resulting in the catheter getting hung-up or hinging.

Whilst different methods of assembling the stent delivery system 40 will suggest themselves to the skilled person, one convenient method is to make a first sub-assembly by necking the distal tip of the inner shaft 45 to decrease its outer diameter dimension, then sliding onto it and positioning in place the pair of marker bands 46. The necking of the distal tip has the advantage of rendering it easier for the operator to slide the marker bands onto the shaft 45 and this feature also provides a contribution to the ease of movement of the inner shaft through bodily lumens when the delivery system is being deployed. Once past the neck, the marker bands 46 are preferably swaged onto their desired positions on the inner shaft 45. Next, the pair of transition sleeves 41 are slid onto the inner shaft 45 and over the necked area. One sleeve 41 is placed over each marker band 46 and fixed or bonded into position by suitable means such as laser welding, heat welding or use of an adhesive. The fixation of the sleeve 41 has the secondary advantage of also fixing the marker band 46 in place, preventing its accidental dislocation during subsequent assembly steps. This completes the first sub-assembly. A second sub-assembly is made independently by cutting a length of balloon from a tube of balloon material, sliding the proximal end of the balloon over the distal end 43a of outer shaft 43 and bonding it into position at proximal balloon bond 48. The final assembly is then made by sliding the first sub-assembly into the second sub-assembly, then bonding the distal end of the balloon to the distal outer surface of the inner shaft to form the distal balloon bonding 47.

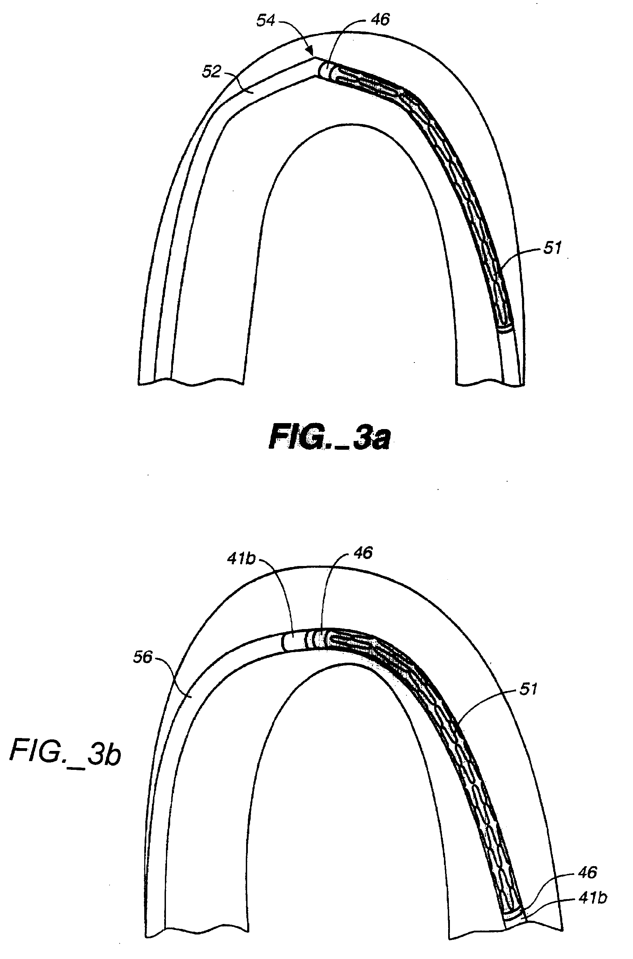

Referring now to Figure 3a, there is shown a view of a stent 51 mounted on a balloon catheter 52 traversing through an acute bend, there being no transition sleeve provided. The balloon catheter 52 is exhibiting what is known by those skilled in the art as hinging at 54. Figure 3b shows a view of a stent 51 mounted on a balloon catheter 56 with a transition sleeve 416 as described above traversing through the same acute bend. The balloon catheter 56 is not exhibiting the hinging effect.

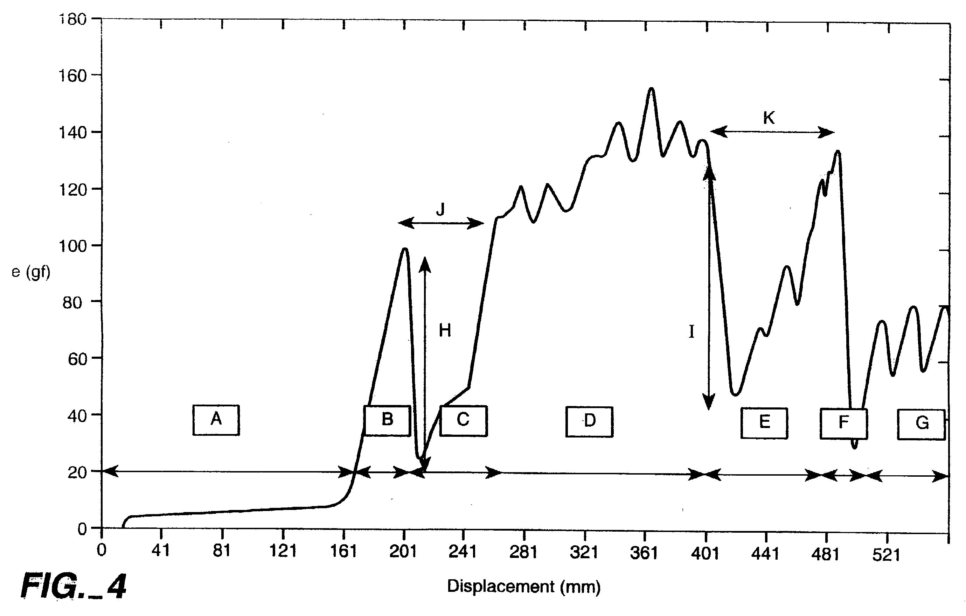

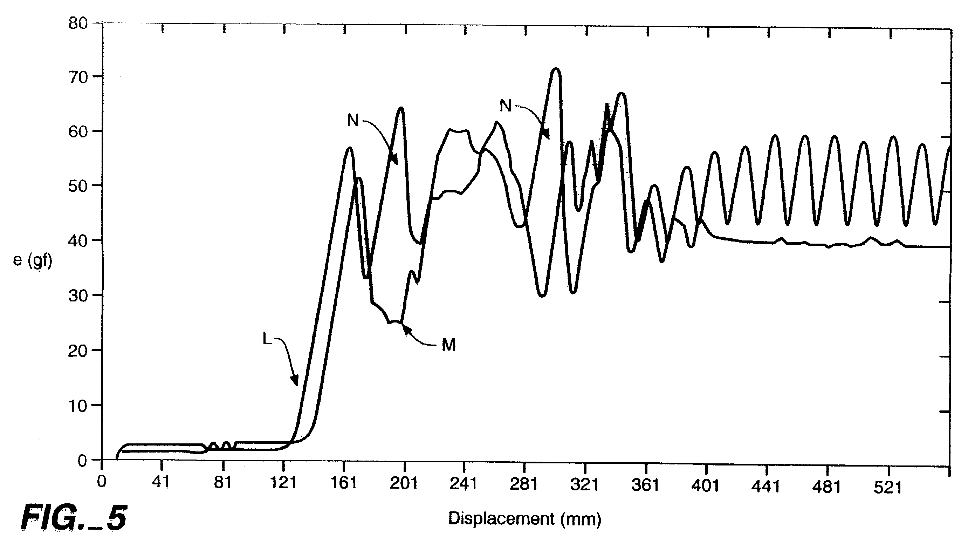

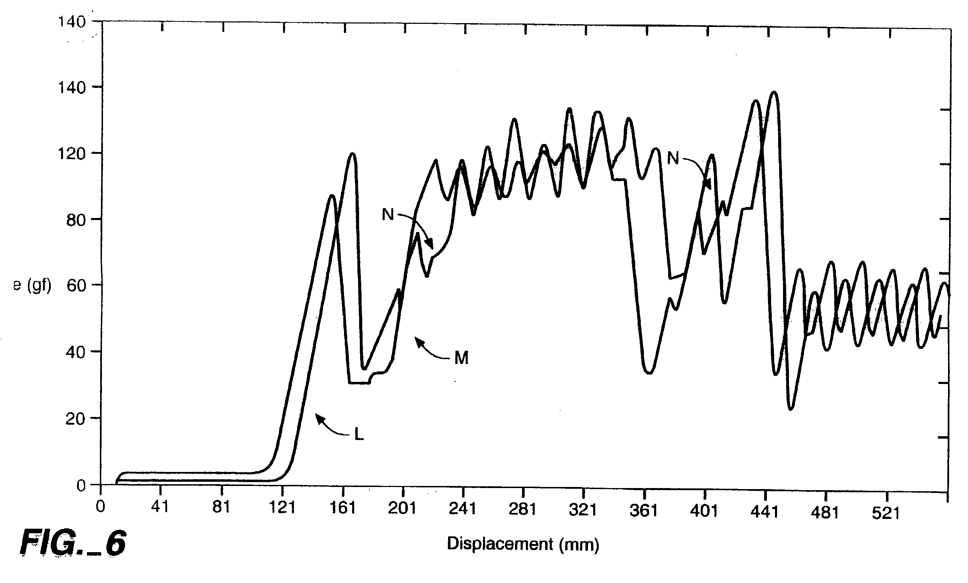

Track testing on catheters with and without transition sleeves located proximal and distal to the stent was undertaken and profiles were derived from the test results. The test involved pushing distal sections of the catheters around a fixed wire tube with a radius of 3mm and a 90° bend. The resistance felt by the attached load cell as the various sections of the catheter track around the bend radius were recorded. Figure 4 shows a typical track profile observed. This track profile of the catheter without transition sleeves has a series of peaks and valleys, Figures 5 and 6 are track profiles for two different sized stent delivery systems. Each figure features a track profile for a same sized stent delivery system with and without transition sleeves provided. There is noticeable smoothing of the track profile for the catheters with transition sleeves. It is important to note that the system track performance does not deteriorate significantly due to the addition of the transition sleeves.

The track profile is broken up in sections A to G along the displacement axis. These sections define the reactive force experienced by the load cell as specific sections of the device track around the bend and are as follows:

A: Wire Movement Value, i.e. the resistance force experienced as the distal section travels along the straight section of wire. This value is used to normalize all the specimens in the test series.

B: Tip Seal Force; the resistance encountered as the portion of the inner shaft 45 which extends distally of the balloon welds, the weld itself and finally the balloon neck travel around the bend.

C: Distal Trough; this part of the profile is where the flexible section of the device between the rigid tip seal weld/balloon neck and the stent tracks around the bend.

D: Mid-Stent Force; approximation of the mean resistance force encountered as the stent tracks around the bend.

E: Proximal Trough; the part of the profile where the flexible section of the device between the rigid stent and proximal balloon weld and balloon neck tracks around the bend.

F: Balloon Bond Force; the resistance encountered as the balloon neck and the balloon weld travel around the bend.

G: Distal Shaft Force; the resistance encountered as the outer and inner distal shafts negotiate the bend, the main resistance contribution coming from the outer shaft.

H: Distal Trough Amplitude = Max. Tip Seal Force Value minus the Min. Distal Trough Force Value.

I: Proximal Trough Amplitude = Max. Balloon Bond Force Value minus the Min. Proximal Trough Force Value.

J: Distal Trough Width

K: Proximal Trough Width

In Figure 5, the stent used is a 2.5mm diameter, 9mm length one and in Figure 6, the stent used is a 4.0 mm diameter, 18mm length one. In each case, the transition sleeve were comprised of 100% 7233 0.0255" x 0.029" (0.6477mm x 0.7366mm) clear Pebax™ extrusion cut to length.

L: With Sleeves

M: Without Sleeves

N: Transition Sleeve Force Peaks

The effect of the transition sleeves can be seen on the graphed track profiles, and the two distinctive peaks in the distal and proximal troughs produced by the areas described. The important contribution of the transition sleeves is effectively to reduce the width and amplitude of the two troughs, thereby smoothing out the track profile between the relatively stiff and flexible regions of the device. The device effectively tracks much smoother to the touch, as opposed to the jumpy track effect experienced with the device without the transition sleeve.

The effect of the sleeves on performance characteristics appears negligible. The system track performance does not deteriorate significantly due to the addition of the transition sleeves. We have found that the track forces in general are slightly higher on shorter, small diameter balloons, while there seems to be improved track with larger longer balloons. This is expected, as the shorter, smaller diameter devices may not have the dimensional capacity to effectively articulate the increased stiffness in a beneficial manner.

Various materials may be selected for the transition sleeve and indeed each "sleeve" may be formed in any way which serves the desired purpose of providing a region of material flanking the distal and proximal ends of the stent which has a stiffness which is intermediate the relatively stiff stent and relatively flexible shaft or shafts and the term "sleeve" as used therein is to be construed accordingly. Thus, whilst the invention has been described particularly above in terms of a pair of transition sleeves located on an inner shaft, it is to be understood that the desired effect may be achieved in many ways including but not limited to:

providing a single transition sleeve, either at the distal or proximal side of the stent or both;

providing a single transition sleeve extending from proximal to distal the stent;

providing the or each transition sleeve on the inner shaft or the balloon;

providing the or each transition sleeve as an area of the inner shaft and/or the balloon and formed integrally therewith, for example, as an area of increased shaft diameter or balloon thickness.

providing the or each transition sleeve in such a way that it combines the roles of providing the desired stiffness transition with the role of providing a visualisation marker, either of a visible or radiopaque nature or preferably both, the sleeve and marker being formed integrally or separately.

It will of course be understood that the invention is not limited to the specific details herein described, which are given by way of example only and that various alterations and modifications may be made without departing from the scope of the invention as defined by the appended claims.

## Claims

1. A vascular catheter (40) comprising:
An elongated tubular member with a proximal shaft portion and a distal shaft portion, the distal shaft section having a first stiffness;
an intravascular device on the distal shaft portion, the distal shaft section having a second stiffness at the intravascular device position; and
a transition member (41,416) on the distal shaft portion bridging the first stiffness and second stiffness.

2. A catheter of claim 1 wherein the transition member comprises a tubular member.

3. A catheter of claim 1 or claim 2 wherein the transition member is positioned on the distal shaft section on at least one side of the intravascular device.

4. A catheter of claim 1 or claim 2 wherein the transition member is positioned on the distal shaft section on both sides of the intravascular device.

5. A catheter as claimed in any of the preceding claims wherein the transition member comprises a radiopaque marker (46).

6. A vascular catheter (40) for performing vascular procedures, the vascular catheter comprising:
an elongated shaft having an elongated inner shaft (45), an elongated outer shaft (43) coaxial with the inner shaft, a proximal shaft section and a distal shaft section;
a means (44) for performing a vascular procedure on the distal shaft section; and
a transition means (41,416) on the distal shaft section adjacent the means for performing a vascular procedure.

7. A vascular catheter of claim 6 wherein the means for performing a vascular procedure is a stent delivery system.

8. A vascular catheter of claim 6 wherein the transition member is a sleeve on the inner shaft of the vascular catheter.

9. A vascular catheter of claim 6 wherein the transition member further comprises a radiopaque marker (46).
